**Europäisches Patentamt**

**European Patent Office**

**Office européen des brevets**

(19)

(11) Publication number: **0 045 605**

**A2**

(12) **EUROPEAN PATENT APPLICATION**

(21) Application number: **81303424.6**

(22) Date of filing: **27.07.81**

(51) Int. Cl.³: **A 61 M 5/16**

(30) Priority: **02.08.80 GB 8025372**

(43) Date of publication of application:
**10.02.82 Bulletin 82/6**

(84) Designated Contracting States:
**BE DE FR IT**

(71) Applicant: Clarke, Jacqueline Margaret
47 Deramore Drive
Belfast Northern Ireland, BT9 5JS(GB)

(72) Inventor: Clarke, Ellis Whiteside
47 Deramore Drive
Belfast Northern Ireland BT9 5JS(GB)

(72) Inventor: Clarke, Jacqueline Margaret
47 Deramore Drive
Belfast Northern Ireland BT9 5JS(GB)

(74) Representative: Wallbank, Roger Wickham et al,
BARKER, BRETTELL & DUNCAN 138 Hagley Road
Edgbaston Birmingham, B16 9PW(GB)

(54) **Needle assemblies for use in enabling gases to enter and to leave liquid containers.**

(57) A needle assembly comprises a tubular needle 10 which is chamfered at one end and has a hollow hub 11, made of a plastics material such as polypropylene, secured to its other end. A filter element 12 is non-detachably bonded to the hub. The filter element comprises a hydrophobic membrane formed with through pores of relatively small size, for example 3 micrometres or 0.5 micrometres. In one method of manufacture the filter element is pressed against the proximal end of the hub which has been previously heated to melt or soften it and is then allowed to solidify again. In use the needle is inserted into a liquid container and permits air or other gas to enter the container as liquid is withdrawn from the container through an outlet. Liquid cannot escape by way of the assembly owing to the hydrophobic nature and small pore-size of the membrane.

FIG.3.

FIG.4.

Croydon Printing Company Ltd.

1

# NEEDLE ASSEMBLIES FOR USE IN ENABLING GASES TO ENTER AND TO LEAVE LIQUID CONTAINERS

This invention relates to needle assemblies for use in enabling gases to enter and to leave liquid containers. The invention is primarily concerned with needle assemblies for use with containers containing liquids that are to be employed for medical purposes. For example, a needle assembly embodying the present invention may be used to enable air to enter a bottle containing a parenteral liquid, such as a saline or nutrient solution, as the liquid gradually flows from the bottle, through a suitable outlet, for infusion into a patient. A needle assembly embodying the present invention may alternatively be used to enable air or gas to leave or enter a vial containing a drug. Drugs that are to be injected into patients are usually supplied in bottles closed with self-sealing diaphragms. A needle assembly embodying the present invention may be used to pierce the diaphragm and enable gas to leave or air to enter so as to bring the pressure in the vial to atmospheric pressure. Then, if water is introduced into the vial to dissolve the drug, gas can escape through the assembly; and when the liquid is withdrawn from the vial with the aid of a syringe, air can enter the vial through the assembly.

A known type of needle assembly for purposes such as these comprises a tubular needle with a hollow support secured to its proximal end, the interior of the support communicating with the bore of the needle, and the bore being provided with filter means for filtering air passing into the needle.

In one kind of needle assembly of that type, the hollow support contains filter means constituted by a mass of fibrous material such as cotton wool. This kind of assembly suffers from the disadvantage that it becomes largely or wholly inoperative if liquid from inside the container comes into contact with the filter means.

In another kind of needle assembly of that type, intended not for these purposes but for filtering liquids passing through the assembly, the hollow support contains a cup-shaped filter element made of sintered metal. Such an assembly would again be unsuitable for the purposes described above as the element would become largely or wholly inoperative if it became wet.

In another type of needle assembly there is provided a tubular needle with a hollow support secured to its proximal end, the support containing a body formed with a restricted passageway extending through it. This does not serve as a filter at all but merely deters the flow of liquid back through the needle when the assembly is in use.

An aim of the present invention is to provide an improved form of needle assembly, which incorporates filter means but in which the difficulty outlined above is overcome or at least reduced.

From one aspect the present invention comprises a needle assembly for use in enabling air or other gas to enter or leave a liquid container, comprising a tubular needle with a hollow support secured to its proximal end, the interior of the support communicating with the bore of the needle, and the support being provided with filter means for filtering air passing into the needle, characterised in that the hollow support comprises a hub in the form of a tubular element of a plastics material, and the filter means comprises a hydrophobic filter element non-detachably bonded to the hub.

As the filter element is hydrophobic it does not become inoperative when liquid touches it. Moreover it can serve as a barrier to prevent liquid escaping from the assembly.

The filter element preferably comprises a membrane formed with through pores. The pores are preferably of a substantially uniform, predetermined size. The pore-size is preferably not greater than 10 micrometres.

When a needle assembly embodying the present invention is in use, it is often the case that the needle is inserted through a diaphragm into a bottle, vial or other container containing a parenteral liquid, and air enters the container through the assembly as liquid leaves the container or is withdrawn from it. In many circumstances the assembly is so positioned that its hub is well below the level of the liquid, so that liquid tends to flow back down the needle into the hub and to lie against the filter element. The element is so selected that under the liquid-pressure likely to be encountered in normal use no liquid escapes from the assembly through the filter element. In this connection it will be appreciated that if even a single drop of liquid were to escape through the element it would be likely to become contaminated, with the result that the liquid inside the container would be likely also to become contaminated if the drop were sucked back into the assembly during the subsequent passage of air into the assembly.

When the assembly is used to enable air to enter an infusion bottle, the maximum pressure likely to be encountered is about 6 pounds per square inch (p.s.i.), that is about 41 $kN/m^2$; to resist the passage of liquid through the filter element under such pressure it is found that a pore-size of about 3 micrometres may be employed. When the assembly is used to enable gas to leave a previously sealed vial containing an aqueous solution or suspension of a drug, and subsequently to allow air to enter the inverted vial as the liquid is withdrawn with a syringe, the maximum pressure likely to be encountered is likely to be considerably greater, and may be about 20 p.s.i. (that is about 138 $kN/m^2$). To resist the passage of liquid through the filter element at such a pressure it is found that a pore-size of about 0.5 micrometres may be used; for example, one suitable type of filter material that is commercially available has a nominal pore-size of 0.45 micrometres.

A reduction in pore-size of a filter element normally results in an increased resistance to the flow of air or other gas through the element. To keep the resistance as low as possible it is therefore desirable, for any particular application, to use a filter element with a pore-size as large as possible, while being small enough to prevent the flow of liquid through the element when in use.

The filter element is preferably situated at or close to that end of the hub further from the needle.

The filter element may be of concave shape, when viewed externally, so as to reduce the likelihood of its being accidentally damaged when in use. When, however, the liquid pressure likely to be encountered in the container, in use, is relatively high, it is preferred to make the filter element of convex shape, when viewed externally, so as the better to resist the pressure of liquid in the assembly. The liquid is of course prevented from passing through the element owing to its hydrophobic nature and its small pore-size.

From another aspect the present invention consists in a method of making a needle assembly for use in enabling air or other gas to enter and to leave a liquid container, characterised in that it comprises the steps of securing a hub to the proximal end of a tubular needle, the hub comprising a tubular element of a plastics material, so that the interior of the hub communicates with the bore of the needle, and bonding in a non-detachable manner a hydrophobic filter element to the hub, either before or after the hub is secured to the needle, the arrangement being such that in use, gas entering the hub for passage through the needle is constrained to pass through the filter element, and the filter element is such that gas can be drawn through it but that liquid cannot escape from the assembly through the filter element.

The filter element may be bonded to the hub by means of an adhesive, but in a preferred method at least part of the hub is made from a thermoplastic material which is heated to cause it to melt or soften, and the filter element is applied to the melted or softened material which is then caused or allowed to set so as to bond the element to the hub.

In the course of manufacture it is preferred to cause air or some other gas to pass through the filter element before it is bonded to the hub to remove any loose material from the inside face of the filter element, that is the face that will be directed towards the interior of the assembly. In a convenient method that passage of air or other gas through the filter element is used temporarily to hold the filter element against a carrier prior to its being transferred to a retainer from which it is transferred to the hub. Further, that passage of air or other gas may also be used to withdraw the filter element from one end of a stack of similar filter elements. The filter element is preferably transferred from the carrier to the retainer by means of a reverse flow of air or other gas.

From another aspect the present invention consists in a needle assembly for use in enabling gases to enter and to leave liquid containers, when made by a method of the kind outlined above.

From yet another aspect the present invention consists in apparatus for carrying out the method outlined above and characterised in that it comprises a magazine for housing a stack of filter elements, a carrier connectable to a vacuum and movable from a position adjacent to an outlet end of the magazine to a retainer which is also connectable to a vacuum, and a needle carriage for carrying a needle with a hub attached to it from a position in which the hub is adjacent to a heating element to a position in which the hub is adjacent to the retainer. In a preferred form of apparatus the carrier and carriage are interconnected so as to be movable in unison.

An embodiment of the invention will now be described in more detail, by way of example, with reference to the accompanying drawings, in which:-

Figure 1 is a plan view of apparatus embodying the present invention and including a partially assembled needle assembly,

Figure 2 is similar to Figure 1 but is to a smaller scale and shows part of the apparatus in a different position,

Figure 3 is a side view to a larger scale than those of Figures 1 and 2 of a needle assembly embodying the present invention, and

Figure 4 is an end view of the needle shown in Figure 3 as viewed from the right of Figure 3.

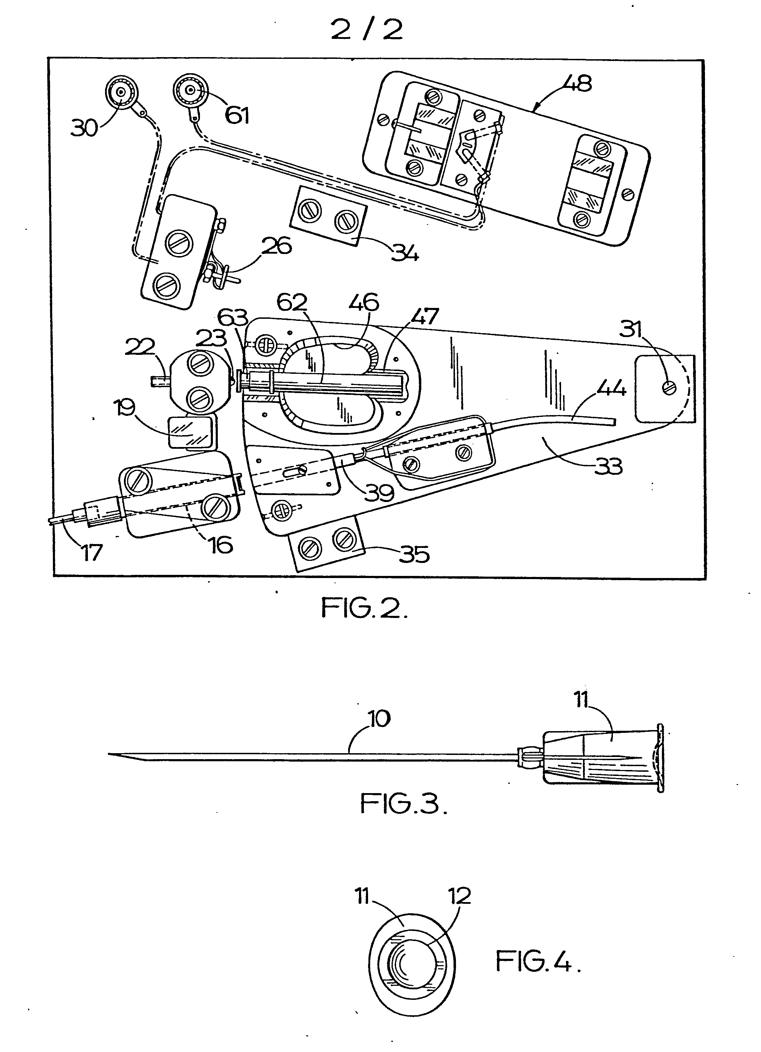

The needle assembly shown in Figures 3 and 4 comprises a needle 10 with a support in the form of a hub 11 secured to its proximal end and a filter element 12 bonded to the hub in a non-detachable manner. The needle and hub are assembled together before the filter element is bonded in place, and conveniently comprise an injection needle unit of a standard kind manufactured for medical or veterinary use in the injection or removal of liquids and of the kind that is chamferred at its distal end. The details of the interconnection between the needle and the hub will therefore not be described here. Suffice it to say that the hub 11 comprises a tubular element made from a thermoplastic plastics material such as polypropylene and that the interior of the hub communicates with the bore of the needle 10. The filter element 12 is bonded to the mouth of the hub 11 at that end of the hub further from the needle. The method in which the filter element is bonded in position is described in detail below. The filter element 12 comprises a disc of filter material of a known kind comprising a film of porous material having a pore-size of about 3 micrometres secured to a support layer consisting of a fine nylon textile fabric. The filter material is of the known kind that has been treated with a substance which makes it hydrophobic.

The central part of the filter element 12 is concave, this reducing the likelihood of the element being accidentally damaged during installation or in use. Suitable filter material is marketed by Gelman Sciences Incorporated, of Ann Arbor, Michigan, U.S.A., under the designation "Hydrophobic Acropore Membrane".

It is the usual practice for injection needle units of the kind described and illustrated to be supplied in tubular protectors or guards. Each protector comprises a tube closed at one end and open at the other. The needle projects axially into the tube from the open end thereof while the hub, which is of slightly tapered shape externally, is held by friction in the mouth of the tube, part of the hub projecting into the tube and part of it projecting outwards, beyond the end of the tube. The needle is supported by the hub in such a manner that it is held away from the side walls and from the end wall of the tube. The protector may be closed by a tubular cap which, when in place, contains the projecting part of the hub and embraces an end part of the protector adjacent to the mouth of the protector. In that case the protector and cap together constitute a container for the unit. Other protectors are not provided with caps, in which case each needle unit in its protector is enclosed within a sealed envelope which constitutes a container. As will become apparent from the description given below of the method of bonding the filter element to the hub, use is made of the tubular protector in carrying out the method. Nevertheless it is to be understood that if the needle and hub unit is manufactured or supplied without such a protector use may be made of a similarly shaped component specially made for use in carrying out that method.

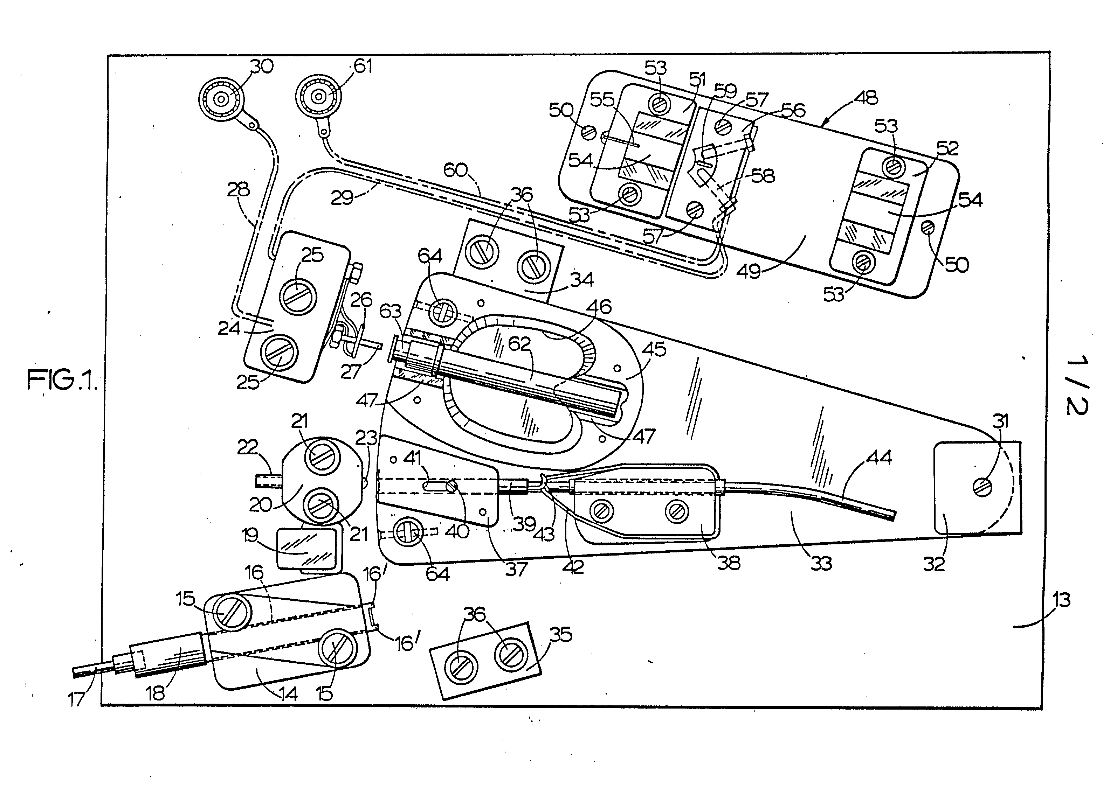

In bonding the filter element to the hub use is made of the apparatus shown in Figures 1 and 2. The apparatus comprises a horizontal base board 13 on which various components are mounted. A block 14 secured to the base board by screws 15 is formed with a horizontal bore housing a tube 16 constituting a magazine for filter elements. A stack of filter elements is inserted into the tube 16, and the inlet end of the tube is connected to a flexible pipe 17 by way of a resilient connection 18 which can readily be detached and replaced as required. In use the pipe 17 is connected to a source of pressurized air which gently urges the stack of filter elements towards the outlet end of the tube 16. The air is filtered to ensure that no solid particles are blown onto the filter elements. In an alternative arrangement (not shown) the tube contains a helical compression spring which urges the stack of elements towards the outlet end of the tube. At that outlet end the tube is formed with three retaining fingers 16' disposed uniformly around the tube. Each of the fingers 16' is inwardly inclined sufficiently far to prevent the filter elements being blown from the tube 16 but not so far as to prevent the element at the adjacent end of the stack from being withdrawn as described below. The position of the tube 16 can be adjusted axially relative to the block 14.

Alongside the block 14 is a mirror 19 disposed at 45° to the horizontal. Next to the mirror 19 is a second block, 20, made of polytetrafluorethylene (PTFE) and secured to the board by screws 21 and having a horizontal bore extending through it. A pipe 22 enters one end of the bore and can be connected to a vacuum (i.e. a source of reduced pressure) as described below. At the other end of the bore there is a metal gauze 23 shaped as a dome of a diameter substantially equal to that of the interior of the hub 11, at the mouth of the hub. The gauze and the end of the pipe to which it is attached constitute a retainer.

Beyond the second block 20 is a third block, 24, secured to the board by screws 25. The third block supports an electric heating element 26 in the shape of a planar spiral disposed in a vertical plane. A metal pin 27 extends axially through the centre of the spiral-shaped element. The ends of the element are connected to electrical leads 28 and 29, the former of which is connected to a terminal post 30 mounted on the base board.

The tube 16, mirror 19, gauze 23 and pin 27 are all directed towards or aligned with a vertical pivot 31 near the other end of the board. The pivot extends through one arm of a bracket 32 mounted on the base board, and through one end part of a plate 33 located between that arm and the base board. The shape of the plate appoximates to that of a sector of a circle, the pivot being at the centre. The plate 33 can be moved to and fro about the axis of the pivot 31, its travel being limited by stops 34 and 35 secured to the base board by screws 36. Rollers 64 are mounted in apertures in the plate, near the arcuate edge thereof, and project a short distance beneath the plate. The presence of the rollers ensures that the plate can be moved smoothly and accurately to and fro between the stops 34 and 35.

A pair of aligned blocks 37 and 38 is secured to the upper face of the plate 33 near one radial edge thereof, and the blocks are formed with axially aligned bores directed radially from the pivot axis. A tube 39, constituting a carrier for filter elements, is mounted for axial movement in the bore through the outer block, 37. A screw 40 projects radially from the tube 39 and enters a slot 41. Engagement between the screw 40 and the ends of the slot 41 limits the axial movement of the tube 39. A resilient band 42 extends around the inner block 38 and over a hook 43 connected to the tube 39 so as to urge the tube into a retracted position, as illustrated in Figure 1. A Bowden cable mechanism (not shown) is connected to the tube 39 so as to enable the tube to be moved from its retracted position to a projecting position, against the action of the band 42. The Bowden cable mechanism is connected to an operating lever or pedal for operation by the user. In one convenient arrangement the operating lever is arranged for operation by lateral movement of one of the user's knees when the user is seated before the base board. The tube 39 is also connected to a flexible pipe 44.

Alongside the blocks 37 and 38 on the plate 33 there is mounted a needle carriage in the form of a plate 45 secured to the plate 33 and formed with a through hole 46, roughly in the shape of a heart, and with grooves 47 in its upper surface, the grooves being aligned with each other and with the pivot axis of the plate 33. The groove nearer the pivot axis, unlike the other groove, terminates short of the edge of the plate 45.

A sealing device 48 is also mounted on the base board 13, though it forms no part of the apparatus described above and could be sited elsewhere or even omitted altogether. The sealing device comprises a base plate 49 secured to the base board by screws 50, with a pair of locating blocks 51 and 52 secured to it by screws 53. Mutually aligned locating grooves 54 are formed in the upper surfaces of the locating blocks. A wire spring 55 projects into one end of the locating groove in the block 51. Between the locating blocks 51 and 52 there is a heater block 56 secured to the base plate 49 by screws 57. Mounted on the heater block 56 is a pair of screws 58 with a resistance element 59 connected between their adjacent ends. A central part of the resistance element is disposed above the level of the heater block and extends in alignment with the locating grooves 54. One of the screws 58 is connected to the electrical lead 29, while the other is connected to one end of an electrical lead 60 of which the other end is connected to a terminal post 61.

. The pipe 22 and the flexible pipe 44 are connected to a two-way air valve having an operating lever or pedal for operation by the user. Where the Bowden cable mechanism is operated by a knee-lever, as described above, the valve may conveniently be operated by a second knee-lever, the knee-levers being operable one by the operator's one knee and the other by the other knee.

The apparatus is used in the following manner. It is assumed, solely for ease of description, that the operator is male; it is of course to be understood that a female operator may equally well use the apparatus. The operator has a supply of preformed needle and hub units. Each unit is mounted in a container comprising a protector with a cap as described above. The operator takes a first container, removes the cap from the protector and lays the cap aside. He then places the protector in the grooves 47 in the plate 45. A protector 62 is shown in this position in Figures 1 and 2. The protector is so orientated that the hub 63 of the needle is adjacent to the arcuate edge of the plate 33. The operator holds a central part of the protector between his fingers and thumb, his fingers and thumb entering the hole 46. The operator urges the protector, as far as it will go, towards the pivot axis of the plate 33, so that the closed end of the protector abuts the end of the groove 47 nearer the pivot axis. The operator, using the protector as a handle, also urges the plate 33 to the position shown in Figure 2, in which it abuts the stop 35. The carrier tube 39 is then aligned axially with the magazine tube 16. The operator operates the Bowden cable mechanism to cause the tube 39 to project and to engage the end filter element in the magazine tube. The operator also operates the air valve so as to connect the pipe 44 to a vacuum. Then the operator operates the Bowden cable mechanism again so that the tube 39 returns to its retracted position; as this occurs the end filter element is withdrawn from between the retaining fingers 16' and is held by air pressure against the end of the carrier tube. As the filter element is porous, air passes through it and removes any loose material from the inside face of the filter element, that is the face that is directed towards the pivot axis and that will be directed towards the interior of the final assembly.

Still using the needle protector 62 as a handle the operator moves the plate 33 away from the stop 35. As the tube 39 passes the mirror 19 the operator, looking down onto the mirror, can see a reflection of the filter element held against the end of the tube and can check that it is held centrally on the end of the tube. The operator continues the movement of the plate 33 until it abuts the stop 34, as shown in Figure 1. He then operates the Bowden cable mechanism to cause the tube 39 to project and to carry the filter element that is held to it into engagement with the metal gauze 23. He next operates the air valve, by means of the lever, so as to disconnect the pipe 44 from the vacuum and to connect the pipe 22 to the vacuum. This completes the transfer of the filter element from the end of the tube 39 to the gauze 23. Finally the operator operates the Bowden cable mechanism again, thus causing the tube 39 to return to its retracted position. While performing these operations for transferring the filter element from the tube 39 to the metal gauze, the operator slides the protector 62 axially along the grooves 47 so that the mouth of the hub 63 approaches the heating element 26. Throughout the operation of the apparatus the terminal posts 30 and 61 are connected to a source of electricity so that the element 26 and the element 59 are both maintained at a dull red heat. Movement of the hub 63 towards the element 26 continues until the pin 27 abuts the inner end of the hub 62. The mouth of the hub is then only a very short distance, typically one millimetre, from the heating element. The operator holds the protector in this position for a short time, typically two seconds, while the end face of the hub, around the mouth, is heated and becomes soft or melts. It is highly desirable for the hub to be very close to the heating element as this leads to the softening or melting of only a thin layer of material of the hub. If the hub were held further from a heater there would be the danger that too great a depth of the hub material would become softened or melted.

When these operations have been performed the operator moves the protector 62 back as far as possible in the grooves 47 towards the pivot axis and swings the plate 33 back to the position shown in Figure 2. He then moves the protector outwards again, away from the pivot axis so that the softened material around the mouth of the hub engages the marginal part of the filter element on the gauze 23. At the same time the operator operates the air valve lever to disconnect the vacuum from the pipe 22. Finally the operator lifts the protector 62 from the grooves 47, thus separating the hub 63 from the block 20 with the filter element now bonded to the hub. As the block 20 is made from PTFE the softened or molten material of the hub does not adhere to it. After removal of the protector and needle from the apparatus the material of the hub that was previously softened or melted sets again quickly without the need for special cooling steps to be taken.

The operator removes the protector, fits the cap to it and places the assembled container in the locating grooves 54 of the sealing device 48, the wire spring 55 ensuring that the cap is held against the protector and that the container abuts that end of the grooves further from the spring. As the operator lowers the container into the grooves, part of the underside of the container and an adjacent part of the cap momentarily engage the heated resistance element 59. The materials from which they are made are softened and locally caused to merge. When the container is removed from the sealing device it is found that the cap is lightly sealed to the protector. This sealing ensures that in use a sharp pull is required to remove the cap from the container. The user can thus distinguish between an unopened container, containing an unused assembly, from a container that has been previously opened and that may therefore contain an assembly that has already been used. It may be remarked here that a needle assembly of the kind described would usually be used only once and would then be discarded.

After putting the sealed container aside the operator takes another container, removes the cap, and places the protector in the grooves 47. He can then repeat the sequence of operations described above, as many times as required. It will be appreciated that in each sequence of operations, when the plate 33 is in the position shown in Figure 2 a fresh filter element can be withdrawn from the magazine tube 16 while the previously withdrawn element is bonded to the associated hub. Likewise, while the plate 33 is in the position shown in Figure 1, the filter element is transferred from the tube 39 to the gauze 23 while the end of the next hub is being heated.

If the protector is of the kind that has no cap, the sealing device 48 is not used.

The finished needle assembly can be used in enabling air to enter an infusion bottle containing a parenteral liquid and having a closure made from a plastics material. A needle connected to tubing leading eventually to a patient is inserted into the bottle through the closure. The needle of the needle assembly is also inserted into the bottle, through the closure, alongside the first needle. The bottle is then suspended from a suitable stand in an inverted position. Parenteral liquid is withdrawn from the bottle in a controlled manner in the usual way, and the liquid leaving the bottle is replaced by air drawn upwards into the bottle, through the needle assembly. It is found that if liquid from the bottle passes into the needle assembly, at any stage, it does not escape from the assembly owing to the nature of the filter element, that is its hydrophobic quality and its small pore-size.

A needle assembly for use in enabling gas or air to enter or leave a vial can be made in a manner very similar to that described above. The filter element, however, would normally be of a smaller pore-size, and may for example have a pore-size of 0.45 micrometres. As mentioned above, the pressure likely to be encountered by the filter element is greater than that likely to be encountered by the element in a needle assembly of the kind used with an infusion bottle. This is, of course, the principal reason for the selection of a filter element with a reduced pore-size. In view of the increased pressure likely to be encountered in use, the filter element is preferably shaped so as to convex, when viewed externally, rather than concave. Further, it is preferably bonded more firmly onto the hub than the concave element described above. To achieve this, the retainer constituted by the gauze 23 and the adjacent end of the tube 22, is modified slightly. The gauze is made concave and is secured to the end of the tube 22, which projects slightly from the block 20. The end of the tube is flared outwards a little. When the heated end of the hub 63 is pushed against the retainer, the flared end of the tube 22 is forced a short way into the hub, the maximum diameter of the end of the tube being substantially equal to the mean of the internal and external diameters of the melted or softened end of the hub. The end of the tube, with the gauze and the filter element retained on the gauze, therefore displace some of the melted or softened material inside the mouth of the hub so that an annular shoulder is formed. When the hub is then withdrawn from the retainer, the margin of the filter element remains on the shoulder, and it is found that the melted or softened material between the filter element and the adjacent end of the hub tends to move radially inwards and to overlap the margin of the element before setting. Thus the margin of the element becomes firmly embedded a short distance inside the mouth of the hub.

## CLAIMS

1. A needle assembly for use in enabling air or other gas to enter or leave a liquid container, comprising a tubular needle with a hollow support secured to its proximal end, the interior of the support communicating with the bore of the needle, and the support being provided with filter means for filtering air passing into the needle, characterised in that the hollow support comprises a hub in the form of a tubular element of a plastics material, and the filter means comprises a hydrophobic filter element non-detachably bonded to the hub.

2. A needle assembly according to claim 1 characterised in that the filter element comprises a membrane formed with through pores of predetermined size.

3. A needle assembly according to claim 2 characterised in that the pore-size is not greater than 10 micrometres.

4. A needle assembly according to claim 3 characterised in that the pore-size is about 3 micrometres.

5. A needle assembly according to claim 3 characterised in that the pore-size is about 0.5 micrometres.

6. A needle assembly according to any one of claims 2 to 5 characterised in that the filter element is situated at or close to that end of the hub further from the needle.

7. A needle assembly according to claim 6 characterised in that the filter element is of concave shape, when viewed externally, so as to reduce the likelihood of its being accidentally damaged when in use.

8. A needle assembly according to claim 6 characterised in that the filter element is of convex shape, when viewed externally, so as the better to resist the pressure of liquid in the assembly.

9. A method of making a needle assembly for use in enabling air or other gas to enter and to leave a liquid container, characterised in that it comprises the steps of securing a hub to the proximal end of a tubular needle, the hub comprising a tubular element of a plastics material, so that the interior of the hub communicates with the bore of the needle, and bonding in a non-detachable manner a hydrophobic filter element to the hub, either before or after the hub is secured to the needle, the arrangement being such that in use, gas entering the hub for passage through the needle is constrained to pass through the filter element, and the filter element is such that gas can be drawn through it but that liquid cannot escape from the assembly through the filter element.

10. A method according to claim 9 characterised in that it includes the step of causing the passage of air or other gas to pass through the filter element before it is bonded to the hub, in order to remove any loose material from the inside face of the element, that is the face which in use is directed towards the interior of the assembly.

11. A method according to claim 10 characterised in that it includes the step of employing said passage of air or other gas to hold temporarily the filter element against a carrier, prior to its being transferred to a retainer from which it is transferred to the hub.

12. A method according to claim 11 characterised in that is includes the step of employing a flow of air or other gas to withdraw the filter element from one end of a stack of similar filter elements, that flow or air or other gas the continuing and causing said passage of air or other gas through the filter element.

13. A method according to either of claims 11 and 12 characterised in that the filter element is transferred from the carrier to the retainer by means of a reverse flow of air or other gas.

14. A method according to any one of claims 9 to 13 characterised in that the hub comprises a thermoplastic material, part of the hub is heated to cause it to melt or soften, and the filter element is applied to the melted or softened material which is then caused or allowed to set so as to bond the filter element to the hub.

15. A needle assembly for use in enabling gases to enter and to leave liquid containers, characterised in that it is made by a method according to any one of claims 9 to 14.

16. Apparatus for carrying out the method claimed in claim 14 comprising a magazine for housing a stack of filter elements, a carrier connectable to a source of reduced pressure and movable from a position adjacent to an outlet end of the magazine to a position adjacent to a retainer which is also connectable to a source of reduced pressure, and a needle carriage for carrying a needle with a hub attached to it from a position in which the hub is adjacent to a heating element, operable to heat the bulb so as to cause part of the hub to melt or become soft, to a position in which the hub is adjacent to the retainer.

17. Apparatus according to claim 16 characterised in that the carrier and carriage are interconnected so as to be movable in unison, so that when either the carrier or the needle hub is in its position adjacent to the retainer, the other is in the other of its positions.

FIG.1.

2 / 2

FIG.2.

FIG.3.

FIG.4.